(19) Europäisches Patentamt
European Patent Office
Office européen des brevets

(11) **EP 1 390 480 B1**

## (12) FASCICULE DE BREVET EUROPEEN

(45) Date de publication et mention
de la délivrance du brevet:
**02.11.2011 Bulletin 2011/44**

(21) Numéro de dépôt: **02738292.8**

(22) Date de dépôt: **31.05.2002**

(51) Int Cl.:
*C12N 9/08* (2006.01)   *C12P 3/00* (2006.01)
*C12N 11/00* (2006.01)   *A61L 2/16* (2006.01)
*A01N 59/24* (2006.01)   *A01N 63/00* (2006.01)
*C12M 1/40* (2006.01)

(86) Numéro de dépôt international:
**PCT/FR2002/001844**

(87) Numéro de publication internationale:
**WO 2002/097076 (05.12.2002 Gazette 2002/49)**

(54) **PROCEDE DE PRODUCTION ENZYMATIQUE D'UN AGENT DE TRAITEMENT A L'ETAT FLUIDE**

VERFAHREN ZUR ENZYMATISCHEN HERSTELLUNG VON EINEM BEHANDLUNGSMITTEL IM FLÜSSIGEN ZUSTAND

METHOD FOR THE ENZYMATIC PRODUCTION OF A CURING AGENT IN ITS FLUID STATE

(84) Etats contractants désignés:
**AT BE CH CY DE DK ES FI FR GB GR IE IT LI LU MC NL PT SE TR**

(30) Priorité: **31.05.2001 FR 0107344**

(43) Date de publication de la demande:
**25.02.2004 Bulletin 2004/09**

(73) Titulaire: **TMI Europe**
**69120 Vaulx en Velin (FR)**

(72) Inventeur: **CASEZ, Hervé**
**F-69480 Pommiers (FR)**

(74) Mandataire: **Bernstein, Claire Jacqueline et al**
**Cabinet Orès**
**36, rue de Saint Pétersbourg**
**75008 Paris (FR)**

(56) Documents cités:
EP-A- 0 397 227   EP-A- 0 518 445
GB-A- 2 209 523   US-A- 4 761 380

• **MARSHALL V M E ET AL: "COMPARISON OF THE ANTI BACTERIAL ACTIVITY OF THE HYPO THIO CYANITE ANION TOWARDS STREPTOCOCCUS-LACTIS AND ESCHERICHIA-COLI" JOURNAL OF GENERAL MICROBIOLOGY, vol. 120, no. 2, 1980, pages 513-516, XP001053150 ISSN: 0022-1287**

• **MORENO M ET AL: "ALCOHOL FERMENTATION IN STRICT ANAEROBIOSIS IN A PLUG FLOW FERMENTOR EFFECT OF CELL RE CYCLING" BIOTECHNOLOGY LETTERS, vol. 1, no. 12, 1979, pages 483-488, XP008009708 ISSN: 0141-5492**
• **KATZBAUER B ET AL: "Classification system for immobilization techniques", BIOPROCESS ENGINEERING, vol. 12, no. 4, 1995, pages 173-179, ISSN: 0178-515X**
• **BOYD S A ET AL: "SELECTIVE EFFECTS OF SMECTITE-ORGANIC COMPLEXES ON THE ACTIVITIES OF IMMOBILIZED ENZYMES", JOURNAL OF MOLECULAR CATALYSIS, vol. 34, no. 1, 1986, pages 1-8, ISSN: 0304-5102**
• **MODI ET AL: 'Lactoperoxidase-catalyzed oxidation of thiocyanate by hydrogen peroxide: 15N nuclear magnetic resonance and optical spectral studies.' BIOCHEMISTRY vol. 30, 1991, pages 118 - 124**
• **TENOVUO ET AL: 'Products of thiocyanate peroxidation: properties and reaction mechanisms' BIOCHIMICA ET BIOPHYSICA ACTA vol. 870, 1986, pages 377 - 384**

Remarques:
Le dossier contient des informations techniques présentées postérieurement au dépôt de la demande et ne figurant pas dans le présent fascicule.

EP 1 390 480 B1

## Description

La présente invention concerne la génération d'un flux concentré et continu d'espèces chimiques oxygénées et de substrats oxydés, en phase liquide, permettant d'obtenir des solutions utilisables par exemple pour le lavage, la décontamination, l'aseptisation de différents produits alimentaires dont l'eau, mais aussi de matériels industriels, ainsi que pour la dépollution et l'assainissement de fluides et pour la préparation de produits alimentaires, pharmaceutiques et cosmétiques.

L'utilisation à cette fin de systèmes enzymatiques antimicrobiens naturels, comme les oxydoréductases, par exemple le système lactoperoxydase est connu et de nombreuses applications sont décrites.

Les propriétés de ce système enzymatique sont notamment étudiées dans "The lactoperoxidase system chemistry and biological significance" (1985) Marcel Dekker, Inc, New York, Chap. 8 pp 143-178.

Ce système antimicrobien lactoperoxydase / thiocyanate / peroxyde d'hydrogène est décrit par exemple dans la demande de brevet EP-A-0397227 et comporte schématiquement trois composantes :

- une enzyme : la lactoperoxydase,
- un substrat oxydable : l'ion thyocyanate ($SCN^-$)
- un donneur d'oxygène : le peroxyde d'hydrogène.

Dans ce système, en milieu liquide, la lactoperoxydase catalyse la réaction d'oxydation du thiocyanate.

En présence de peroxyde d'hydrogène en quantité suffisante et dans des conditions de pH correctes, la réaction d'oxydation se poursuit vers des dérivés oxyacides encore plus oxydés.

Les espèces chimiques oxygénées seules ou en mélange, obtenues sont de façon non limitative, l'ion hypothiocyanite $OSCN^-$, les ions $O_2SCN^-$ et $O_3SCN^-$, les anions superoxyde $O_2^-$ et trioxyde $O_3^-$, l'ion hydroxyle $OH^-$, l'oxyde nitrique $NO$, le trioxyde dinitré $N_2O_3$, le dioxyde nitré $NO_2$, le peroxynitrite $ONO_2$, l'hydroperoxynitrite $ONHO_2$, le dioxyde de soufre $SO_2$, le trioxyde de soufre $SO_3$, l'acide sulfureux $HSO_3$ et l'acide hypochloreux $HOCl$.

Les espèces chimiques oxygénées précitées sont connues pour leurs effets bactériostatique et bactéricide, notamment vis à vis de nombreux microorganismes tels que bactéries, par exemple les *Pseudomonae,* les *Enterobacteriaceae,* comme *E. Coli, Salmonella,* les *Listeria* ou *Campylobacter,* les formes sporulées et les protozoaires, les virus, les levures ou les champignons.

Ce système antimicrobien peut ainsi être utilisé pour la décontamination par action des espèces chimiques oxygénées obtenues, notamment les ions thiocyanates oxydés comme $OSCN^-$, $O_2SCN^-$, $O_3SCN^-$, qui sont susceptibles d'interagir avec les composants des membranes cellulaires ou d'oxyder des polluants chimiques.

En présence d'un substrat fournisseur de peroxyde d'hydrogène qui peut être le peroxyde d'hydrogène lui-même ou par exemple un peroxyde métallique ou un système enzymatique complémentaire producteur de peroxyde d'hydrogène, ce système enzymatique complémentaire étant par exemple une oxydoréductase avec un substrat oxydable et d'oxygène comme le système glucose/glucose-oxydase en milieu aqueux, l'utilisation du système complet est possible et les propriétés réactionnelles d'un tel système antimicrobien comportent schématiquement trois étapes :

- la production de peroxyde d'hydrogène par le fournisseur de peroxyde d'hydrogène,
- la réaction d'oxydation du thiocyanate,
- la décontamination par actions des espèces chimiques oxygénées obtenues.

De nombreuses applications sont décrites.

Par exemple, selon WO-A-8707838 un procédé est décrit pour conditionner sous forme sèche une composition antibactérienne contenant de la lactoperoxydase, du thiocyanate et un donneur d'oxygène natif en vue de son utilisation ultérieure.

On connaît de US-C-5403450 l'utilisation d'installations, dans lesquelles les oxydoréductases sont immobilisées, pour convertir des substances susceptibles d'être oxydées contenues à titre de pollution dans de l'eau.

JP58152486 enseigne, par exemple l'immobilisation d'enzymes sur des particules de polymères pour une utilisation répétée.

Dans la publication « Comparison of the anti-bacterial activity of the hypothiocyabite anion towards streptococcus-lactis and escherichia-coli », Journal of General Microbiology, vol. 120, No.2, 1980, pages 513-516, Marshall et al. décrivent un procédé de préparation d'un agent de traitement, à l'état fluide, comprenant de l'OSCN- séparé de la lactoperoxydase. Cependant, l'OSCN- ainsi préparé est lié à la Sepharose, et n'est donc pas à l'état libre, et présente une stabilité d'environ 30 minutes.

On connaît aussi des systèmes d'immobilisation d'enzymes ou de systèmes enzymatiques sur des parois de réacteurs, sur des films, des billes, et autres supports comportant une surface spécifique importante.

Toutes ces utilisations ne sont cependant pas satisfaisantes, en raison de la consommation excessive d'enzymes qu'elles entraînent :

- soit en raison de leur conditionnement, par exemple sous forme sèche, qui implique une utilisation par saupoudrage des produits à traiter, donc des quantités d'enzymes proportionnelles aux surfaces et aux volumes de produits à traiter,
- soit en raison des rendements faibles de réaction des enzymes immobilisées,
- soit en raison de la rapide dégradation des espèces chimiques oxygénées qui sont générées.

Le procédé selon l'invention permet de résoudre l'ensemble des inconvénients précités en ce qu'il permet de produire un agent de traitement à l'état fluide, par exemple liquide, comprenant à l'état libre au moins une espèce chimique oxygénée, stable, avec un rendement important et une endurance importante.

La présente invention a donc pour objet un procédé de production enzymatique d'un agent de traitement à l'état liquide comprenant à l'état libre l'ion hypothiocyanite (OSCN$^-$), dans lequel :

a) on forme et on met en mouvement un bain réactionnel aqueux comprenant :

- un substrat oxydable en phase aqueuse choisi comme étant le thiocyanate de sodium (NaSCN) ou le thiocyanate de potassium (KSCN),
- un donneur d'oxygène choisi comme étant le peroxyde d'hydrogène,
- un agent coagulant choisi parmi les sels d'aluminium ou les sels de fer,
- un agent épaississant choisi parmi les argiles, le kaolin, la silice ou les silicates,
- un agent floculant choisi parmi les floculants polymères anioniques ou cationiques, les polysaccharides, les hétéropolysaccharides de type anionique ou les polyacrylamines, celui-ci étant introduit dans le bain réactionnel aqueux après l'étape de coagulation, et
- un agent de catalyse enzymatique en phase solide et divisée distribué dans ledit bain, ledit agent de catalyse étant la lactoperoxydase,

pour former des agrégats de particules solides inertes vis-à-vis de l'agent de catalyse enzymatique, lesdits agrégats comprenant ou incorporant, à l'état libre, c'est-à-dire un état de mise en suspension dans les agrégats sans formation de liaison ionique ou covalente entre l'agent de catalyse et les agents coagulant et floculant, ledit agent de catalyse, et lesdits agrégats étant distribués dans le bain réactionnel aqueux,

b) on sépare le bain réactionnel aqueux, en une fraction enrichie en en agrégat, et une fraction dépourvue d'agrégat, à partir de laquelle l'agent de traitement est obtenu.

Le procédé de production enzymatique selon l'invention consiste donc à mettre en contact:

- un agent de catalyse enzymatique choisi comme étant la lactoperoxydase,
- un substrat oxydable en phase aqueuse susceptible d'être oxydé par action d'un donneur d'oxygène, ledit donneur d'oxygène étant le peroxyde d'hydrogène, par catalyse par ledit agent de catalyse enzymatique, en générant l'ion hypothiocyanite (OSCN$^-$) à l'état libre, ledit substrat oxydable étant le thiocyanate de sodium (NaSCN) ou le thiocyanate de potassium (KSCN),
- un agent coagulant choisi parmi les sels d'aluminium ou les sels de fer,
- un agent épaississant choisi parmi les argiles, le kaolin, la silice ou les silicates, et
- un agent floculant choisi parmi les floculants polymères anioniques ou cationiques, les polysaccharides, les hétéropolysaccharides de type anionique ou les polyacrylamines,

et permet d'obtenir un agent de traitement à l'état liquide, comprenant à l'état libre l'ion hypothiocyanite (OSCN$^-$). L'agent de traitement ainsi obtenu est stable pendant plus de 10 heures.

On entend par une espèce chimique oxygénée à l'état libre, une espèce chimique à l'état ionique dont la constante de dissociation, au pH de la solution obtenue permet le déplacement de l'équilibre de la réaction de dissociation vers l'existence à l'état libre de ladite espèce chimique oxygénée.

On entend par agrégat toute formulation permettant de maintenir en phase solide et divisée mais à l'état libre dans le milieu réactionnel l'agent de catalyse enzymatique par addition de coagulant et de floculant en présence d'épaississeur permettant leur isolement dudit milieu réactionnel en fin de réaction et leur recyclage.

Dans le cadre de la présente invention, la formation des agrégats est effectuée par addition successive d'un coagulant et d'un floculant, on a ainsi une étape de coagulation qui précède l'étape de floculation.

L'agent coagulant est choisi parmi les sels d'aluminium ou de fer, de préférence comme étant le sulfate d'aluminium, le chlorure d'aluminium, l'aluminate de sodium, le polyhydroxychlorure d'aluminium, le polyhydroxysulfate d'aluminium, le polyhydroxychlorosulfate d'aluminium, le polychlorosulfate basique d'aluminium, le polyhydroxychlorosilicate d'aluminium, le fluorosulfate d'aluminium, le sulfate ferreux, le sulfate ferrique, le chlorure ferrique, le chlorosulfate ferrique, la soude, ou des homopolymères de chlorure de diméthyl diallyl ammonium.

Avantageusement les coagulants sont additionnés au milieu réactionnel, dans des proportions variant de 0,1 $10^{-6}$ à 10 g/l de milieu réactionnel.

Avantageusement les floculants sont additionnés au milieu réactionnel, dans des proportions variant de 0,1 $10^{-6}$ à 10 g/l de milieu réactionnel.

L'agent épaississant utilisé dans le cadre de la présente invention est choisi parmi les argiles, le kaolin, la silice ou des silicates. Cet agent épaississant peut être introduit dans le bain simultanément à l'introduction de l'agent de catalyse enzymatique ou après formation du bain réactionnel aqueux.

Avantageusement l'agent épaississant est additionné au milieu réactionnel, dans des proportions variant de 0,1 à 100 g/l de milieu réactionnel.

L'invention présente les variantes suivantes :

- on introduit dans le bain réactionnel aqueux le substrat oxydable en phase aqueuse.
- on introduit dans le bain réactionnel l'agent de catalyse enzymatique à l'état de phase solide et divisée ou en phase liquide.
- on évacue du bain réactionnel l'agent de catalyse enzymatique.
- le procédé est pratiqué en continu, ou de manière discontinue.
- on introduit dans le bain un agent épaississant.
- on introduit dans le bain un agent correcteur de pH.
- on introduit dans le bain un donneur d'oxygène sous forme d'un système enzymatique complémentaire qui produit du peroxyde d'hydrogène, et l'agent de catalyse enzymatique comprend, outre la lactoperoxydase, une enzyme du type oxydoréductase.

L'ensemble de réactifs est mis en oeuvre par introduction dans le réacteur à raison de 0,2 à 10 g/l de volume utile.

Dans des modes de réalisations particuliers, le pH du milieu de dispersion de l'agrégat, peut être stabilisé ou corrigé par addition d'un agent correcteur de pH, qui sera choisi parmi les acides ou bases minérales ou organiques.

Avantageusement les enzymes sont additionnées au milieu réactionnel, dans des proportions variant de 0,02 à 10 g/l de milieu réactionnel.

Avantageusement les substrats enzymatiques associés sont additionnés au milieu réactionnel, dans des proportions variant de 0,05 mM à 15 mM par litre de milieu réactionnel.

Le donneur d'oxygène selon la présente invention est le peroxyde d'hydrogène. De manière générale tout composé chimique susceptible de produire du peroxyde d'hydrogène peut être utilisé.

Avantageusement le peroxyde d'hydrogène est additionné au milieu réactionnel, dans des proportions variant de 0,05 mM à 15 mM par litre de milieu réactionnel.

Lorsque le donneur d'oxygène est sous forme d'un système enzymatique complémentaire qui produit du peroxyde d'oxygène celui-ci comprend un substrat oxydable et une enzyme par exemple du type oxydoréductase, spécifique de ce substrat. Ainsi lorsque les systèmes enzymatiques utilisés sont des oxydoréductases, pour que l'étape de production du peroxyde d'hydrogène soit réalisée, un substrat oxydable seul ou en association est additionné au milieu, ce substrat sera choisi parmi les substances comme le glucose, le lactose, la xanthine.

On citera à titre d'exemple les systèmes enzymatiques suivants, glucose oxydase/glucose, galactose oxydase/galactose, urate oxydase/urate, choline oxydase/choline, glycine oxydase/glycine, glutamate oxydase/glutamate, alcool oxydase/alcool.

Ces systèmes enzymatiques sont capables en présence d'oxygène et d'eau de produire du peroxyde d'hydrogène qui sera utilisé comme donneur d'oxygène dans le système enzymatique du procédé selon l'invention.

Alternativement ce donneur d'oxygène peut être choisi parmi les microorganismes comme *Streptococcus* et/ou *Lactobacillus* qui peuvent produire du peroxyde d'hydrogène.

La mise en oeuvre de l'étape b) du procédé selon l'invention, c'est à dire l'étape de séparation du bain réactionnel aqueux, en une fraction enrichie en agent de catalyse enzymatique en phase solide et divisée et une fraction dépourvue en dit agent de catalyse, à partir de laquelle l'agent de traitement est obtenu est réalisée par des moyens de séparation et récupération des agrégats ou des émulsions. Parmi les moyens mis en oeuvre on citera à titre d'exemple les moyens classiquement utilisés comme la décantation, la flottation, la centrifugation pour les émulsions et la sédimentation, filtration frontale ou tangentielle ou une centrifugation pour les floculats ou coagulats et/ou la séparation cyclonique.

Le procédé selon l'invention permet de produire de grandes quantités de solution d'agent de traitement selon l'invention, possédant des propriétés biocides, utilisables pour le nettoyage, lavage et désinfection des matériels, machines, des outils, des textiles, des récipients et tuyauteries et des locaux et établissements industriels agroalimentaires ou des établissements hospitaliers et de soins. Le procédé permet également la préparation de solutions destinées à la formulation de produits cosmétiques et pharmaceutiques destinés à la santé humaine et/ou animale et de produits alimentaires.

Il permet également de produire des solutions de lavage pour la décontamination de surface des produits alimentaires par exemple fruits, légumes, feuilles mais aussi produits animaux.

Ces solutions biocides pourront être utilisées par bain, pulvérisation, injection, brumisation.

Il sera également possible d'utiliser ces solutions comme constituant d'une composition de produit, par exemple eau de reconstitution de jus de fruit après déshydratation, concentration.

Le procédé permet également d'aseptiser et purifier des eaux destinées à la production d'eaux de boissons pour la consommation humaine ou animale, ou des eaux thermales, des eaux de piscines, de bains.

Le procédé selon l'invention permet également le traitement des eaux polluées, eaux usées ou effluents industriels, voire effluents gazeux par circulation dans le liquide.

Les contaminants chimiques par exemple nitrates ou phosphates sont ainsi oxydés, et les polluants organiques également, en fonction des débits et des concentrations en oxydoréductase, la dégradation pourra être totale ou partielle.

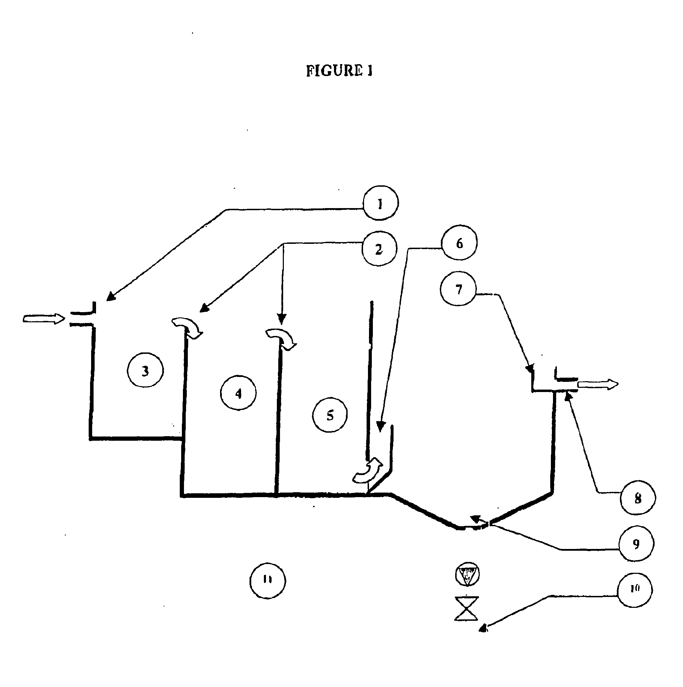

La mise en oeuvre du procédé selon l'invention peut être effectuée dans un réacteur (Figure 1) constitué d'une cuve compartimentée qui peut être close en partie ou en totalité, métallique ou en matériau synthétique dotée d'un orifice de chargement (1) et de surverses et/ou de cloisons siphoïdes, permettant le passage d'un compartiment à l'autre (2).

Le réacteur comporte trois ou quatre compartiments dont deux ou trois sont soumis à une agitation permanente :

- le premier (3) est destiné à recevoir l'agent de catalyse enzymatique, le coagulant et l'agent épaississant, il est soumis à une agitation à vitesse rapide,
- le second (4) reçoit le floculant et optionnellement le correcteur de pH, il est soumis à une agitation à vitesse lente,
- le troisième où l'apport de substrat oxydable est effectué, ainsi que l'apport en donneur d'oxygène, est le siège de la réaction enzymatique souhaitée et est également soumis à une agitation à vitesse lente,
- dans le quatrième compartiment, la séparation du bain réactionnel aqueux, en une fraction enrichie en agent de catalyse enzymatique en phase solide et divisée, et une fraction dépourvue en dit agent de catalyse, à partir de laquelle l'agent de traitement est obtenu, est effectuée sur un sédimenteur lamellaire qui comporte une alimentation basse (6), une surverse (7) reliée à l'orifice de sortie placée juste en dessous du fil de l'eau (8), un point bas d'extraction dynamique des matières solides sédimentées (9) en vue de leur évacuation (10) ou de leur reprise en vue d'un recyclage (11).

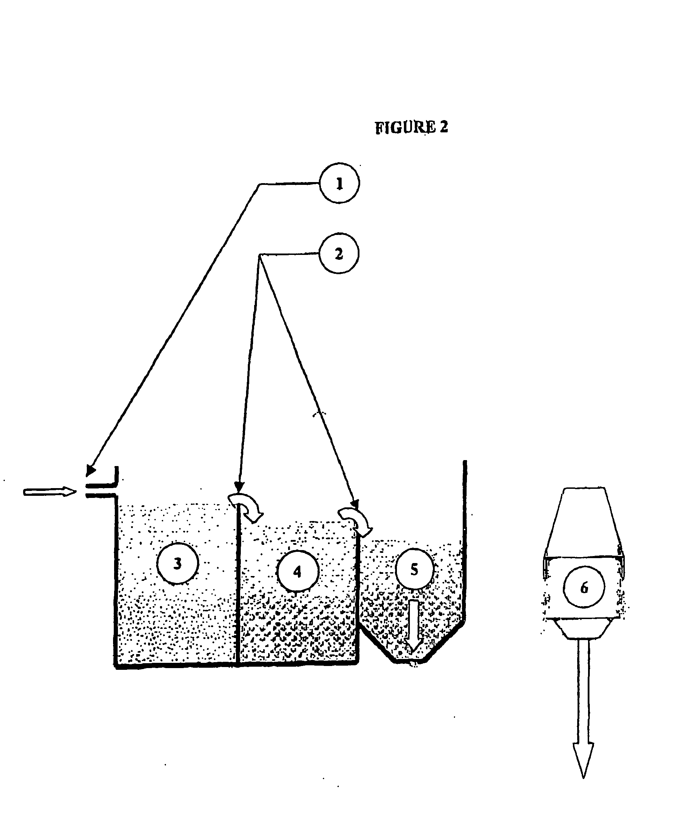

Dans une variante de réalisation, la mise en oeuvre du procédé selon l'invention est effectuée dans un réacteur (Figure 2) constitué d'une cuve compartimentée qui peut être close en partie ou en totalité, métallique ou en matériau synthétique dotée d'un orifice de chargement (1) de surverses permettant le passage d'un compartiment à l'autre comportant trois compartiments dont les deux premiers sont soumis à une agitation permanente.

- le premier dans lequel est introduit l'agent de catalyse enzymatique et l'agent émulsifiant est soumis à une agitation rapide,
- le second dans lequel l'apport de substrat oxydable est effectué et qui est le siège de la réaction enzymatique souhaitée est agité à vitesse lente ;
- le troisième compartiment est un pot de reprise (5) qui permet le pompage en continu de l'émulsion, de la solution comprenant au moins une espèce chimique oxygénée à l'état libre et des substrats résiduels vers une unité de séparation qui peut être un coalesceur, un flottateur, une centrifugeuse (6), un filtre ou un cyclone.

Par application du procédé selon l'invention à la production de solution d'espèces chimiques oxygénées en solution par la mise en oeuvre sous forme de floculat de la lactoperoxydase, en présence de 0.2 à 0.5 mM, de $H_2O_2$ et 0.4 à 1 mM de KSCN, on obtient une solution aqueuse qui contient entre 0.05 et 0.35 mM de d'espèces chimiques oxygénées.

Cette solution utilisée pour le lavage et la décontamination de salades par douches et bains successifs à 10°C permettent une réduction significative (2 à 5 Log en moyenne) de la population des contaminants bactériens comme les *Pseudomonas* ($10^5$ CFU/ml) et une réduction significative de 1 à 2 Log sur les *Listeria* ($10^5$ CFU/ml) par rapport aux salades témoins non traitées, avec un temps de contact de l'ordre de 10 minutes.

Exemple

Dans un réacteur tel que décrit à la Figure 2, 0.25 g/l de lactoperoxydase sont introduits simultanément à 10 g/l d'argile et 0.55 ml de coagulant, après agitation et passage dans le compartiment réactionnel, des quantités variables de KSCN et de $H_2O_2$ sont introduites selon le tableau 1 ci-dessous.

Tableau 1

|  | 1 | 2 | 3 | 4 |
|---|---|---|---|---|
| **KSCN (mM)** | 0.5 | 0.6 | 0.7 | 0.8 |
| **$H_2O_2$ (mM)** | 0.3 | 0.4 | 0.5 | 0.6 |

Après réaction des prélèvements sont effectués pour mesurer l'activité enzymatique et le taux d'espèces chimiques libres obtenu.

Les ions hypothiocyanites ($OSCN^-$) sont capables de réagir avec les groupements sulfhydryls d'une molécule d'acide (5,5'-dithiobis) 2-nitrobenzoique préalablement réduite en présence d'un excès de borohydrure de sodium.

Cette molécule réduite absorbe la lumière à une longueur d'onde de 412 nm.

Lorsque les ions hypothiocyanites ($OSCN^-$) oxydent les groupements sulfhydryls, l'absorbance à 412 nm diminue proportionnellement à la quantité d'ions présents dans l'échantillon, ce qui permet de les quantifier.

L'ion thiocyanate ($SCN^-$) est capable de réagir avec FeCl3 en milieu acide, pour former un produit coloré dont la quantité, proportionnelle à celle du thiocyanate présent, est mesurable par photométrie à une longueur d'onde de 450 nm.

La mesure de la quantité de thiocyanate contenu dans un échantillon nécessite la réalisation d'une gamme d'étalonnage.

Tableau 2 :

|  |  | 1 | 2 | 3 | 4 |
|---|---|---|---|---|---|
| **$OSCN^-$** | **$ADO_{412nm}$** | 0.855 1/2 | 0.999$^{1/2}$ | 1.250 1/2 | 1.320 1/2 |
|  | **[OSCN]mM** | 0.190 | 0.220 | 0.275 | 0.290 |
| **$SCN^-$ Initial** | **$DO_{450nm}$** | 0.242 | 0.252 | 0.279 | 0.310 |
|  | **[SCN]mM** | 0.67 | 0.70 | 0.77 | 0.86 |
| **$SCN^-$ résiduel** | **$DO_{450nm}$** | 0.184 | 0.186 | 0.210 | 0.237 |
|  | **[SCN]mM** | 0.51 | 0.52 | 0.58 | 0.66 |
| **pH** |  | 6.66 | 6.62 | 6.63 | 6.59 |
| **Qualité coagulation** |  | 3/5 | 3/5 | 3/5 | 2.5/5 |

L' activité enzymatique initiale du réacteur est contrôlée dans l'essai n°4. Celle-ci est réalisée à partir de 50$\mu$l de coagulat et correspond à :

$$\Delta DO_{405nm} / 10 \text{ secondes} = 1{,}023$$

L'activité enzymatique est mesurée à l'aide d'un substrat chromogènique le 2-2' Azino-bis ou ABTS par colorimétrie à 405 nm.

La lactoperoxydase catalyse l'oxydation de l'ABTS en présence de peroxyde d'hydrogène. La molécule d'ABTS oxydée présente la caractéristique d'absorber à 405 nm, ce qui permet de mesurer l'activité d'une solution enzymatique en suivant la quantité d'ABTS oxydée produite (proportionnelle à la $DO_{405nm}$ d'après la loi de Beer Lambert) par unité de temps, ceci en mesurant en continu l'absorbance à 405 nm

*Suivi de la stabilité de la solution*

On effectue un suivi de l'évolution de la concentration en ion hypothiocyanite OSCN- dans les solutions en sortie de réacteur par les méthodes décrites ci-dessus.

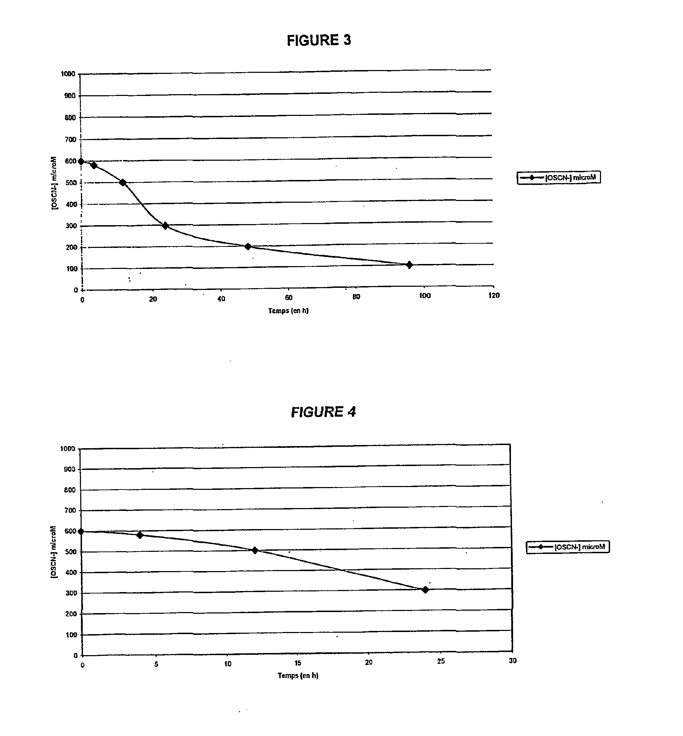

Les résultats obtenus sont illustrés sur les figures 3 et 4, qui représente la courbe de l'évolution de la concentration en ion hypothiocyanite OSCN-, dans l'eau de sortie de réacteur sur une période de 1 jour (Figure 4) et 4 jours (Figure 3).

Les résultats obtenus montrent (voir Figure 4) qu'après 10 heures la concentration de OSCN- passe de 600mM à 500mM, et qu'après 20 heures cette concentration n'a chutée que de 50 %.

Sur une période de 4 jours, on obtient (voir Figure 3) une courbe qui démontre une concentration résiduelle en ion hypothiocyanite OSCN⁻, après 80 heures, égale à 16 % de la concentration initiale.

**Revendications**

1. Procédé de production enzymatique d'un agent de traitement à l'état liquide comprenant à l'état libre l'ion hypothiocyanite (OSCN⁻),
   dans lequel :

   a) on forme et on met en mouvement un bain réactionnel aqueux comprenant :

   - un substrat oxydable en phase aqueuse choisi comme étant le thiocyanate de sodium (NaSCN) ou le thiocyanate de potassium (KSCN),
   - un donneur d'oxygène choisi comme étant le peroxyde d'hydrogène,
   - un agent coagulant choisi parmi les sels d'aluminium ou les sels de fer,
   - un agent épaississant choisi parmi les argiles, le kaolin, la silice ou les silicates,
   - un agent floculant choisi parmi les floculants polymères anioniques ou cationiques, les polysaccharides, les hétéropolysaccharides de type anionique ou les polyacrylamines, celui-ci étant introduit dans le bain réactionnel aqueux après l'étape de coagulation, et
   - un agent de catalyse enzymatique en phase solide et divisée distribué dans ledit bain, ledit agent de catalyse étant la lactoperoxydase,

   pour former des agrégats de particules solides inertes vis-à-vis de l'agent de catalyse enzymatique, lesdits agrégats comprenant ou incorporant, à l'état libre, c'est-à-dire un état de mise en suspension dans les agrégats sans formation de liaison ionique ou covalente entre l'agent de catalyse et les agents coagulant et floculant, ledit agent de catalyse, et lesdits agrégats étant distribués dans le bain réactionnel aqueux,
   b) on sépare le bain réactionnel aqueux, en une fraction enrichie en en agrégat, et une fraction dépourvue d'agrégat, à partir de laquelle l'agent de traitement est obtenu.

2. Procédé selon la revendication 1, **caractérisé en ce qu'**on introduit dans le bain réactionnel l'agent de catalyse enzymatique à l'état de phase solide et divisée.

3. Procédé selon la revendication 1, **caractérisé en ce qu'**on introduit dans le bain réactionnel l'agent de catalyse enzymatique en phase liquide.

4. Procédé selon l'une des revendications 1 à 3, **caractérisé en ce qu'**on introduit dans le bain réactionnel aqueux le substrat oxydable en phase aqueuse.

5. Procédé selon l'une des revendications 1 à 4, **caractérisé en ce qu'**on introduit dans le bain réactionnel aqueux le donneur d'oxygène en phase aqueuse.

6. Procédé selon l'une des revendications 1 à 5, **caractérisé en ce qu'**on évacue du bain réactionnel l'agent de catalyse enzymatique.

7. Procédé selon l'une des revendications 1 à 6, **caractérisé en ce qu'**il est pratiqué en continu, ou de manière discontinue.

8. Procédé selon l'une des revendications 1 à 7, **caractérisé en ce que** l'agent coagulant est choisi parmi le sulfate d'aluminium, le chlorure d'aluminium, l'aluminate de sodium, le polyhydroxychlorure d'aluminium, le polyhydroxysulfate d'aluminium, le polyhydroxychlorosulfate d'aluminium, le polychlorosulfate basique d'aluminium, le polyhydroxychlorosilicate d'aluminium, le fluorosulfate d'aluminium, le sulfate ferreux, le sulfate ferrique, le chlorure ferrique et le chlorosulfate ferrique.

9. Procédé selon l'une des revendications 1 à 8, **caractérisé en ce que** l'on introduit de $0,1.10^{-6}$ à 10 g/l d'agent coagulant.

**10.** Procédé selon l'une des revendications 1 à 9, **caractérisé en ce que** l'on introduit de 0,1 à 100 g/l d'agent épaississant.

**11.** Procédé selon l'une des revendications 1 à 10, **caractérisé en ce qu'**on introduit de $0,1.10^{-6}$ à 10 g/l de l'agent floculant.

**12.** Procédé selon l'une des revendications 1 à 11, **caractérisé en ce qu'**on introduit dans le bain un agent correcteur de pH.

**13.** Procédé selon l'une des revendications 1 à 12, **caractérisé en ce qu'**on introduit dans le bain un donneur d'oxygène sous forme d'un système enzymatique complémentaire qui produit du peroxyde d'hydrogène, et l'agent de catalyse enzymatique comprend, outre la lactoperoxydase, une enzyme du type oxydoréductase.

**Claims**

**1.** Enzymatic process for the production of a treatment agent in liquid form comprising the hypothiocyanite ion ($OSCN^-$) in the free state,
wherein:

   a) an aqueous reaction bath is formed and set in motion, said bath comprising:

   • an oxidisable substrate in the aqueous phase selected to be sodium thiocyanate (NaSCN) or potassium thiocyanate (KSCN),
   • an oxygen donor selected to be hydrogen peroxide,
   • a coagulating agent selected from among the aluminium salts or the iron salts,
   • a thickening agent selected from among the clays, kaolin, silica or the silicates,
   • a flocculating agent selected from among the anionic or cationic polymeric flocculants, the polysaccharides, the anionic heteropolysaccharides or the polyacrylamines, said flocculating agent being added to the aqueous reaction bath after the coagulation step, and
   • an enzymatic catalysing agent in solid and divided phase distributed in said bath, said catalysing agent being lactoperoxidase,

   to form aggregates of solid particles that are inert to the enzymatic catalysing agent, the said aggregates comprising or incorporating the said catalysing agent in free form, i.e. in a state of suspension in the aggregates without the formation of any ionic or covalent bonds between the catalysing agent and the coagulating and flocculating agents, and the said aggregates being distributed in the aqueous reaction bath,
   b) the aqueous reaction bath is separated into an aggregate-enriched fraction, and an aggregate-depleted fraction from which the treatment agent is obtained.

**2.** Process according to claim 1, **characterised in that** the enzymatic catalysing agent is added to the reaction bath in the solid and divided phase.

**3.** Process according to claim 1, **characterised in that** the enzymatic catalysing agent is added to the reaction bath in the liquid phase.

**4.** Process according to one of claims 1 to 3, **characterised in that** the oxidisable substrate is added to the aqueous reaction bath in the aqueous phase.

**5.** Process according to one of claims 1 to 4, **characterised in that** the oxygen donor is added to the aqueous reaction bath in the aqueous phase.

**6.** Process according to one of claims 1 to 5, **characterised in that** the enzymatic catalysing agent is evacuated from the reaction bath.

**7.** Process according to one of claims 1 to 6, **characterised in that** it is carried out continuously or discontinuously.

**8.** Process according to one of claims 1 to 7, **characterised in that** the coagulating agent is selected from among aluminium sulphate, aluminium chloride, sodium aluminate, aluminium polyhydroxychloride, aluminium polyhydroxysulphate, aluminium polyhydroxychlorosulphate, basic aluminium polychlorosulphate, aluminium polyhydroxychlorosilicate, aluminium fluorosulphate, ferrous sulphate, ferric sulphate, ferric chloride and ferric chlorosulphate.

**9.** Process according to one of claims 1 to 8, **characterised in that** from $0.1.10^{-6}$ to 10 g/l of coagulating agent are added.

**10.** Process according to one of claims 1 to 9, **characterised in that** 0.1 to 100 g/l of thickening agent are added.

**11.** Process according to one of claims 1 to 10, **characterised in that** $0.1.10^{-6}$ to 10 g/l of flocculating agent are added.

**12.** Process according to one of claims 1 to 11, **characterised in that** a pH correcting agent is added to the bath.

**13.** Process according to one of claims 1 to 12, **characterised in that** an oxygen donor in the form of a complementary enzymatic system which produces hydrogen peroxide is added to the bath, and the enzymatic catalysing agent comprises, besides lactoperoxidase, an enzyme of the oxidoreductase type.


**Patentansprüche**

**1.** Verfahren zur enzymatischen Herstellung eines Behandlungsmittels im flüssigen Zustand, das das Hypothiocyanition (OSCN⁻) im freien Zustand umfasst, in dem

a) man ein wässriges Reaktionsbad herstellt und in Bewegung bringt, das umfasst:

- ein oxidierbares Substrat in wässriger Phase, ausgewählt aus Natriumthiocyanat (NaSCN) und Kaliumthiocyanat (KSCN),
- einen Sauerstoffdonor wie zum Beispiel Wasserstoffperoxid,
- ein Koagulationsmittel, ausgewählt aus Aluminiumsalzen und Eisensalzen,
- ein Verdickungsmittel, ausgewählt aus Tonen, Kaolin, Siliciumdioxid, Silikaten,
- ein Flockungsmittel, ausgewählt aus anionischen oder kationischen polymeren Flockungsmitteln, Polysachariden, Heteropolysachariden des anionischen Typs und Polyacrylaminen, wobei dieses nach der Koagulationsstufe in das wässrige Reaktionsbad eingeführt wird, und
- ein enzymatisches Katalysemittel in fester und fein verteilter Phase, verteilt in dem Bad, wobei das Katalysemittel Lactoperoxidase ist,

um feste gegenüber dem enzymatischen Katalysemittel inerte Partikelaggregate zu bilden, wobei die Aggregate in freiem Zustand, das heißt in suspendiertem Zustand in den Aggregaten ohne ionische oder kovalente Bindung zwischen dem Katalysemittel und dem Coagulationsmittel und Flockungsmittel, das Katalysemittel umfassen oder eingearbeitet haben, wobei die Aggregate in dem wässrigen Reaktionsbad verteilt sind,

b) man das wässrige Reaktionsbad in eine Fraktion, die an Aggregat angereichert ist, und eine Fraktion, die frei von Aggregat ist, aus der das Behandlungsmittel erhalten wird, auftrennt.

**2.** Verfahren gemäß Anspruch 1, **dadurch gekennzeichnet, dass** man das enzymatische Katalysemittel im Zustand einer festen und fein verteilten Phase in das Reaktionsbad einführt.

**3.** Verfahren gemäß Anspruch 1, **dadurch gekennzeichnet, dass** man das enzymatische Katalysemittel in flüssiger Phase in das Reaktionsbad einführt.

**4.** Verfahren gemäß einem der Ansprüche 1 bis 3, **dadurch gekennzeichnet, dass** man das oxidierbare Substrat in wässriger Phase in das wässrige Reaktionsbad einführt.

**5.** Verfahren gemäß einem der Ansprüche 1 bis 4, **dadurch gekennzeichnet, dass** man den Sauerstoffdonor in wässriger Phase in das wässrige Reaktionsbad einführt.

**6.** Verfahren gemäß einem der Ansprüche 1 bis 5, **dadurch gekennzeichnet, dass** man das enzymatische Katalysemittel aus dem Reaktionsbad unter Vakuum abzieht.

**7.** Verfahren gemäß einem der Ansprüche 1 bis 6, **dadurch gekennzeichnet, dass** es kontinuierlich oder in diskontinuierlicher Art durchgeführt wird.

8. Verfahren gemäß einem der Ansprüche 1 bis 7, **dadurch gekennzeichnet, dass** das Koagulationsmittel aus Aluminiumsulfat, Aluminiumchlorid, Natriumaluminat, Aluminiumpolyhydroxychlorid, Aluminiumpolyhydroxysulfat, Aluminiumhydroxychlorsulfat, basischem Aluminiumpolychlorsulfat, Aluminiumpolyhydroxychlorsilikat, Aluminiumfluorsulfat, Eisen(II)-sulfat, Eisen(III)-sulfat, Eisen(III)-chlorid und Eisen(III)-chlorsulfat ausgewählt wird.

9. Verfahren gemäß einem der Ansprüche 1 bis 8, **dadurch gekennzeichnet, dass** man $0,1 \cdot 10^{-6}$ bis 10 g/l Koagulationsmittel einführt.

10. Verfahren gemäß einem der Ansprüche 1 bis 9, **dadurch gekennzeichnet, dass** man 0,1 bis 100 g/l Verdickungsmittel einführt.

11. Verfahren gemäß einem der Ansprüche 1 bis 10, **dadurch gekennzeichnet, dass** man $1 \cdot 10^{-6}$ bis 10 g/l Flockungsmittel einführt.

12. Verfahren gemäß einem der Ansprüche 1 bis 11, **dadurch gekennzeichnet, dass** man ein pH-Einstellmittel in das Bad einführt.

13. Verfahren gemäß einem der Ansprüche 1 bis 12, **dadurch gekennzeichnet, dass** man einen Sauerstoffdonor in Form eines komplementären enzymatischen Systems, das Wasserstoffperoxid produziert, einführt und das enzymatische Katalysemittel außer Lactoperoxidase ein Enzym des Oxidoreductasetyps umfasst.

FIGURE 1

FIGURE 2

## FIGURE 3

## FIGURE 4

## RÉFÉRENCES CITÉES DANS LA DESCRIPTION

**Documents brevets cités dans la description**

- EP 0397227 A
- WO 8707838 A
- US 5403450 C
- JP 58152486 B

**Littérature non-brevet citée dans la description**

- The lactoperoxidase system chemistry and biological significance. Marcel Dekker, Inc, 1985, 143-178
- **MARSHALL.** Comparison of the anti-bacterial activity of the hypothiocyabite anion towards streptococcus-lactis and escherichia-coli. *Journal of General Microbiology,* 1980, vol. 120 (2), 513-516